# EUROPÄISCHE PATENTANMELDUNG

(11) **EP 0 528 393 A1**
(43) Veröffentlichungstag der Anmeldung: **24.02.1993**
(21) Anmeldenummer: 92113971.3
(22) Anmeldetag: 17.08.1992
(51) Int. Cl.: A61N 1/36

(54) **Implantierbares medizinisches Gerät und Verfahren zu seiner Herstellung**

(30) Priorität: 20.08.1991 DE 4127533
(71) Anmelder: Pacesetter AB, S-171 95 Solna (SE)
(72) Erfinder: Slettenmark, Bruno, S-175 60 Järfälla (SE); Strandberg, Hans, S-172 36 Sundbyberg (SE); Brand, Paul, S-198 00 Balsta (SE)
(74) Vertreter: Lettström, Richard Wilhelm

(57) **Zusammenfassung**

Bekannte implantierbare medizinische Geräte wie z.B. Herzschrittmacher weisen ein verschweißtes Kapselgehäuse auf, das mit einem Gasgemisch aus Helium und Argon gefüllt ist, wobei Helium zum Aufspüren von undichten Stellen in dem Kapselgehäuse dient.

Zur Verbesserung der elektrischen Isolationseigenschaften wird das Kapselgehäuse anstelle von Argon mit einem Isoliergas, vorzugsweise Stickstoff, Schwefelhexafluorid, Kohlendioxyd oder Luft gefüllt. Das medizinische Gerät ist dabei vorzugsweise ein Defibrillator.

## Beschreibung

Die Erfindung betrifft ein implantierbares medizinisches Gerät mit einem verschweißten Kapselgehäuse, das mit einem Gasgemisch aus Helium und einer anderen inerten Gaskomponente gefüllt ist. Die Erfindung betrifft ferner ein Verfahren zur Herstellung eines solchen Geräts.

Bei bekannten Herzschrittmachern ist die Herzschrittmacherelektronik in einem hermetisch abgeschlossenen Kapselgehäuse angeordnet, das mit einer Gasmischung aus Helium und Argon gefüllt ist. Wie beispielsweise aus der US-A-4 174 424 bekannt ist, ist Helium aufgrund seiner extrem kleinen Moleküle zum Aufspüren von undichten Stellen in dem Kapselgehäuse besonders geeignet. Argon wird dagegen vorzugsweise als Schutzgas beim Verschweißen des Kapselgehäuses verwendet (US-A-4 041 956).

Die bekannte Helium-Argon-Gasmischung weist jedoch relativ schlechte elektrische Isolationseigenschaften auf.

Der Erfindung liegt daher die Aufgabe zugrunde, die elektrische Isolierung bei verkapselten medizinischen Geräten zu verbessern.

Zur Lösung der Aufgabe ist erfindungsgemäß vorgesehen, daß bei dem implantierbaren medizinischen Gerät der eingangs angegebenen Art die inerte Gaskomponente ein Isoliergas oder Isoliergasgemisch ist. Als Isoliergas kommen insbesondere mehratomige Gase mit großer Elektronenaffinität und hoher Ionisationsenergie infrage. Dabei besteht die Gaskomponente vorzugsweise aus Stickstoff, Schwefelhexafluorid, Kohlendioxid, Luft, Sauerstoff oder halogenisierten Kohlenwasserstoffen, wie z.B. Freone. Während für die bekannte Gasmischung aus 20% Helium und 80% Argon eine elektrische Durchschlagsfestigkeit von nur 1,3 kV/mm festgestellt wurde, konnte für eine Mischung aus 20% Helium und 80% Stickstoff eine Durchschlagsfestigkeit von mehr als 2,5 kV/mm gemessen werden. Damit verbunden ist der Vorteil, daß ein kompakter Aufbau des implantierbaren Geräts möglich ist, weil der Abstand zwischen den Leitungen und elektrischen Komponenten innerhalb des Geräts verringert werden kann. Durch die Verwendung von mehratomigen Isoliergasen, also Gasen mit großen Molekülen, wird außerdem in vorteilhafter Weise im Fall von mikrofeinen Undichtigkeiten im Kapselgehäuse die Diffusionsgeschwindigkeit für das Isoliergas oder -gasgemisch durch die undichten Stellen hindurch verringert, während das Helium aufgrund seiner kleinen Moleküle das Aufspüren solcher undichten Stellen ermöglicht.

Aus der JP-A-55 099 713 ist ein Metallfilmkondensator mit einem Kapselgehäuse bekannt, das mit einem Isoliergas wie Schwefelhexafluorid, Stickstoff oder Kohlendioxid gefüllt ist. Aus der FR-A-2 648 275 ist ein Kapselgehäuse für Höchstfrequenz-Schaltungskreise bekannt, dessen Teile in einer Helium-Stickstoff-Atmosphäre zusammengeschweißt werden. Keine dieser beiden Schriften betrifft jedoch ein implantierbares medizinisches Gerät.

Dadurch, daß bei dem erfindungsgemäßen Gerät in dem Kapselgehäuse ein Gasüberdruck herrscht, wird in vorteilhafter Weise die freie Weglänge der Elektronen verringert und damit die Isolationseigenschaften verbessert.

Im Rahmen der Erfindung ist weiterhin vorgesehen, daß das implantierbare medizinische Gerät vorzugsweise ein Defibrillator ist, bei dem sich die verbesserte Isolierung aufgrund der von dem Defibrillator erzeugten Hochspannungsimpulse als besonders vorteilhaft erweist.

Insbesondere kann bei unverändertem Aufbau des Defibrillators, also bei unveränderter Packungsdichte der elektrischen Komponenten, die elektrische Spannung erhöht werden.

In bezug auf das eingangs genannte Verfahren zur Herstellung eines implantierbaren medizinischen Gerätes wird die angegebene Aufgabe dadurch gelöst, daß zunächst Teile des das Gerät nach außen hin abdichtenden Kapselgehäuses in einem Vakuum oder einer Schutzgasatmosphäre miteinander verschweißt werden, daß das Innere des Kapselgehäuses durch eine Öffnung in dem Kapselgehäuse mit einem Gasgemisch aus Helium und dem Isoliergas bzw. Isoliergasgemisch gefüllt wird und daß anschließend die Öffnung verschlossen wird.

## Patentansprüche

1. Implantierbares medizinisches Gerät mit einem verschweißten Kapselgehäuse, das mit einem Gasgemisch aus Helium und einer inerten Gaskomponente gefüllt ist, **dadurch gekennzeichnet**, daß die inerte Gaskomponente ein Isoliergas oder Isoliergasgemisch ist.

2. Implantierbares medizinisches Gerät nach Anspruch 1, **dadurch gekennzeichnet**, daß die Gaskomponente aus Stickstoff besteht.

3. Implantierbares medizinisches Gerät nach Anspruch 1, **dadurch gekennzeichnet**, daß die Gaskomponente aus Schwefelhexafluorid besteht.

4. Implantierbares medizinisches Gerät nach Anspruch 1, **dadurch gekennzeichnet**, daß die Gaskomponente aus Kohlendioxid besteht.

5. Implantierbares medizinisches Gerät nach Anspruch 1, **dadurch gekennzeichnet**, daß die Gaskomponente aus Luft besteht.

6. Implantierbares medizinisches Gerät nach Anspruch 1, **dadurch gekennzeichnet**, daß die Gaskomponente aus Sauerstoff besteht.

7. Implantierbares medizinisches Gerät nach Anspruch 1, **dadurch gekennzeichnet**, daß die Gaskomponente aus halogenisierten Kohlenwasserstoffen besteht.

8. Implantierbares medizinisches Gerät nach einem der vorangehenden Ansprüche, **dadurch gekennzeichnet**, daß in dem Kapselgehäuse ein Gasüberdruck herrscht.

9. Implantierbares medizinisches Gerät nach einem der vorangehenden Ansprüche, **dadurch gekennzeichnet**, daß das Gerät ein Defibrillator ist.

10. Verfahren zum Herstellen eines implantierbaren medizinischen Geräts, **dadurch gekennzeichnet**, daß Teile eines das Gerät nach außen hin abdichtenden Kapselgehäuses in einem Vakuum oder einer Schutzgasatmosphäre miteinander verschweißt werden, daß das Innere des Kapselgehäuses durch eine Öffnung in dem Kapselgehäuse hindurch mit einem Gasgemisch aus Helium und einem Isoliergas gefüllt wird, und daß die Öffnung anschließend verschlossen wird.
